# EUROPEAN PATENT SPECIFICATION

(11) **EP 2 065 016 B1**
(45) Date of publication and mention of the grant of the patent: **16.04.2014**
(21) Application number: 08013214.5
(22) Date of filing: 23.07.2008
(51) Int. Cl.: A61F 2/44, A61B 17/70, A61B 17/80, A61F 2/00, A61F 2/30

(54) **Trans-vertebral and intra-vertebral plate and fusion cage device for spinal interbody fusion**
Transvertebrale und intravertebrale Platte und Fusionskäfigvorrichtung zur spinalen Zwischenwirbelfusion
Plaque trans-vertébrale et intra-vertébrale et dispositif de cage de fusion pour la spondylodèse intercorporelle

(30) Priority: 27.11.2007 TW 96144897
(43) Date of publication of application: 03.06.2009
(73) Proprietor: A-Spine Asia Co., Ltd., Taipei City 114 (TW)
(72) Inventor: Lin, Jin-Fu, Taipei City 100 (TW)
(74) Representative: Meyer, Thorsten

(56) References cited:
- FR-A- 2 727 005
- FR-A- 2 880 795
- US-A- 5 591 235
- US-A1- 2004 034 353
- US-A1- 2005 021 029

## Description

The present invention relates to spinal fusion, and more particularly to a trans-vertebral and intra-vertebral plate and a fusion cage with a slot for the plate. The cage with a slot for the plate is inserted into the interbody space and the trans-vertebral and intra-vertebral structure is implanted inside two adjacent vertebral bodies through the slot of the cage, to provide intervertebral stability by limiting or neutralizing intervertebral movement in order to enhance the fusion, specifically for a device which utilizes a trans-vertebral and intra-vertebral plate and a fusion cage element.

Spinal interbody fusion is a commonly performed procedure for degenerative disorders. To achieve a successful fusion, a spinal fixation device is usually used to control intervertebral movement, and at present the spinal fixation devices generally use bone screws as the basic elements and the screws are attached to a rod or a plate outside of the spinal column to constitute a complete fixation system. The screw based fixation systems can be implanted to the spinal column from posterior, anterior or lateral side and they have been proved in biomechanical tests to be capable of controlling six-way of intervertebral movement, including axial rotation, lateral bending, and flexion and extension. Take posterior inserted bone screw spinal fixation system for instance, the implantation method includes dissecting back muscles until posterior bony elements of the spinal column are exposed. Four screws are then threaded through the pedicles into two adjacent vertebral bodies, and connected two plates or rods on both sides.

The shortcomings of the screw based fixation systems are three folds. First, the inherent biomechanical weaknesses of the bone screw include: the contact area of the bone screw is small and about one-tenth of its circumferential diameter in any direction of intervertebral movement; and the weight-loading stress of the screws is transferred to and concentrated at the screw-rod or screw-plate junction, and thus loosened screws inside the vertebral body or broken screws at the screw-rod junction results are not uncommon. Second, during implanting the screw fixation devices into the spinal column, surrounding soft tissues may be injured, and anterior inserted systems may injure the great vessels and posterior inserted systems may jeopardize the nerves. Third, the screw fixation devices are bulky and protruded outside the spinal column that they may irritate surrounding soft tissues and cause discomfort to patients. In addition, the screw fixation devices require complicated assembling procedures, such as taping the bone, inserting the screws and tightening the assembly.

Since early 1990, interbody fusion cage has been popularized until today and are used to maintain intervertebral height and to enhance fusion. The cage can be inserted from posterior, posterolateral, anterior or lateral trajectory. Bone graft is put inside the cage and fusion will occur within an expected time frame. The so-called stand-alone cage uses a fusion cage without any supplemented posterior or anterior fixation device. The biomechanical weakness of the stand-alone cage is that the cage can control flexion and lateral bending movement, but it is less effective in controlling extension and axial rotation movement. Therefore, intervertebral movement is not fully under controlled by using the stand alone cage, and the fusion rate is reported suboptimal. To obtain stability which is a prerequisite for successful fusion, a supplemented screw based spinal fixation device is recommended.

In summary, to achieve a successful interbody fusion, screw based spinal fixation devices are used to control six-way of the intervertebral movement, in conjunction with a fusion cage. The screw based fixation devices require complicated assembling procedure and may be hazardous to the human body.

US 5,591,235 (The preamble of claim 1 is based on this document) discloses a spinal fixation device comprising an intervertebral fusion cage with a vertical slot through which a rod may pass, bone screws to be buried into vertebrae, which bone screws each accommodate a locking cap to hold the rod inside a vertebra.

From FR 2 880 795 a trans-vertebral and intra-vertebral hook and fusion cage device for spinal interbody fusion is known which comprises a plate or a S-shaped hook element and a intervertebral fusion cage member having a slot passing through the fusion cage from one surface to another, both surfaces facing adjacent vertebrae, which slot accomodates said plate or said S-shaped hook. The slot is larger than the plate or than the S-shaped hook allowing the plate or hook to swivel within the slot. To accommodate the plate or the S-shaped hook in the vertebrae holes are foreseen in the surfaces of the vertebrae facing towards each other. To connect the plate or the S-shaped hook within the vertebrae, also bone screws are used.

From FR 2 727 005 a trans-vertebral and intra-vertebral plate and fusion cage device for spinal interbody fusion is known which comprises a plate element and a intervertebral fusion cage member having a slot at one side of its four ends to accomodate said plate. Plate and fusion cage are connected with screws.

The present invention improves upon known screw based spinal fixation devices, which are bulky, complicated in design and handling, for the purpose of limiting intervertebral movement. In a basic form of embodiment, the present invention is a plate device, which is trans-vertebral and intra-vertebral and coupled to a rectangular cage in which there is a slot to accommodate the plate. After inserting the cage into the intervertebral space, one of the longer ends of the plate is placed through the slot of the cage, and perpendicularly facing cortical bones of two adjacent vertebral bodies. The plate device is then hammered and penetrating the cortical bones from lateral or anterior side of the two adjacent vertebral bodies, and the plate device is buried inside the vertebral bodies. The purpose of the trans-vertebral and intra-vertebral plate device coupled with a fusion cage is to neutralize or limit intervertebral movement, to achieve three-dimensional spinal fixation and enhance fusion. The trans-vertebral and intra-vertebral plate and the cage fixation device are intra-vertebral and inter-vertebral respectively, so that the device is zero profile. The operating process of the trans-vertebral and intra-vertebral plate and the fusion cage with accommodating slot requires only a few steps of: inserting the cage, making a bone cut through the slot of the cage, and hammering the plate through the bone cut. Compared to current screw based fixation devices, the present invention is extremely simple to operate, uses low volume of metal, and zero profile.

The above and other objects, features and advantages of the present invention will become apparent from the following detailed description taken with the accompanying drawing.
- Fig. 1: is a schematic perspective view of the transvertebral and intra-vertebral plate and the fusion cage with a slot for the plate, one of the longer ends of the plate is thinned, and the surface of the plate is perforated, and a rectangular cage with a vertical slot at its lateral side to accommodate the plate, in accordance with the present invention;
- Fig. 2: is a side view of the laterally inserted cage and the trans-vertebral and intra-vertebral plate, in a spine column, in accordance with the present invention;
- Fig. 3: is a front view of the laterally inserted cage seated between two adjacent vertebrae and the trans-vertebral and intra-vertebral plate has been inserted through the slot of the cage from the lat- eral side of the vertebral bodies and incorporated with the cage;
- Fig. 4: is a schematic perspective view of the second preferred embodiment of the trans-vertebral and intravertebral plate fixation device with both of the distal ends of the plate bended and with a side hole at the lateral center side of the plate to accommodate a screw to be integrated with the fusion cage;
- Fig. 5: is a side view of the trans-vertebral and intravertebral plate fixation device with bended ends implanted inside the two adjacent vertebrae and integrated with a lumbar fusion cage, the plate and cage are fixed using a screw through the side hole of the plate;
- Fig. 6: is a front view of anteriorly inserted cage and a bended trans-vertebral and intra-vertebral plate, and they are integrated using a screw through the center screw hole of the plate; and
- Fig. 7: is a front view of anteriorly inserted cage and a straight trans-vertebral and intra-vertebral plate, which is obliquely inserted into two adjacent vertebrae, and the cage has an oblique slot to accommodate the plate; the plate and the cage are fixed using a screw, the same as in Fig. 6.

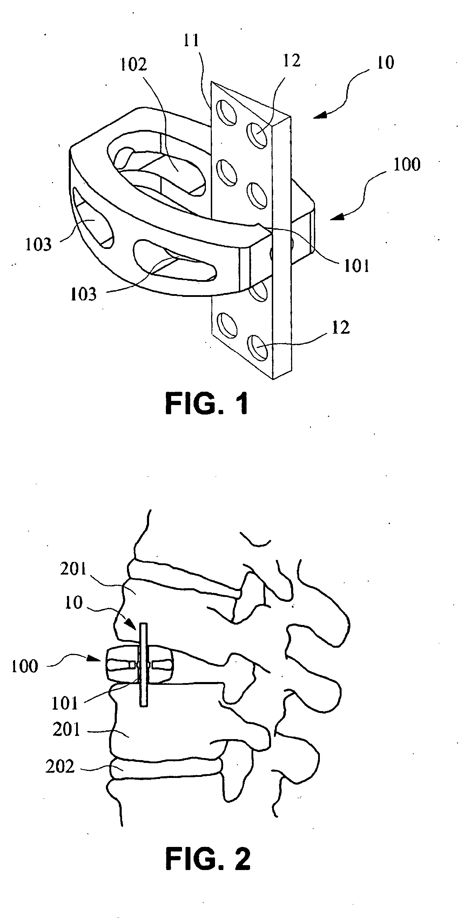

**Fig. 1** shows a preferred embodiment of the present invention which comprises a trans-vertebral and intra-vertebral plate (**10**) and a rectangular cage (**100**) with a vertical slot (**101**) to accommodate the plate (**10**). The trans-vertebral and intra-vertebral plate (**10**) has one of its longer ends thinned (**11**) for the purpose of ease of penetrating into two adjacent vertebral bodies, and the thinned end is facing the cortical bones of the vertebral bodies during insertion. The surface of the plate is perforated (**12**) to facilitate bone ingrowth through the holes (**12**). The rectangular cage (**100**) has a vertical slot (**101**) on its lateral end to accommodate the plate (**10**), and the cage also has holes (**103**) to facilitate bone ingrowth. The plate and the cage can be made of any biocompatible materials such as titanium, carbon fiber, polyether ether ketone (PEEK), and with tensile strength strong enough to resist fatigue failure. The surfaces of the cage and the plate can be serrated to increase contact area with the bones. The plate and the cage in **Fig. 1** are two independent structures and they also can be manufactured into one integrated device. The plate also can be used independently with any brand of cage in the market as long as the plate and the cage are suitable in the same intervertebral space.

**Fig. 2** is a side view of the present invention implanted inside the spinal column from the lateral side of the spinal column. After inserting the cage (**100**) into the intervertebral space (**202**) from the lateral side of the spinal column, the trans-vertebral and intra-vertebral plate (**10**) is placed through the slot (**101**) of the cage and then hammered into adjacent vertebral bodies (**201**) and buried the plate inside the vertebral bodies.

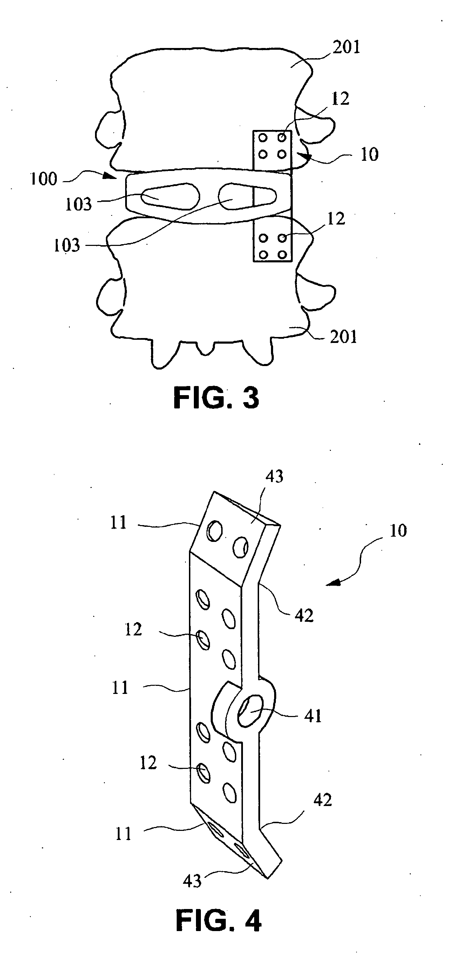

**Fig. 3** is a front view of the present invention implanted inside the spinal column from the lateral side of the spinal column, the same approach as in **Fig. 1****.** The trans-vertebral and intra-vertebral plate (**10**) and the fusion cage (**100**) each plays its own role of limiting, or neutralizing the intervertebral movement. There are six ways of intervertebral movement, including flexion, extension, lateral bending, and axial rotation. When the cage and the plate are inserted from the lateral side of the spinal column (as shown in **Fig. 2** **and** **3**), into two adjacent vertebral bodies, the cage controls flexion and lateral bending of intervertebral movement, and the plate controls extension and axial rotation. Therefore the six ways of intervertebral movement are under controlled, or neutralized by simultaneously using the cage and the trans-vertebral and intra-vertebral plate, to achieve the same purpose of three-dimensional fixation, as bone screw based spinal fixation systems.

**Fig. 4** shows the second preferred embodiment of the trans-vertebral and intra-vertebral plate in accordance to present invention. Both of the two distal ends of the plate (**10**) are bended to a certain angle (**42**) to improve bone purchase and the fixation capability of the plate inside the vertebral bodies. The surfaces (43) of the bended ends also have perforated holes (**12**) for bone ingrowth. At the center of outer end of the plate, there is a screw hole (**41**) to accommodate a screw, and the cage has a corresponding threaded screw hole for the screw. The plate and the cage are then fixed using a screw.

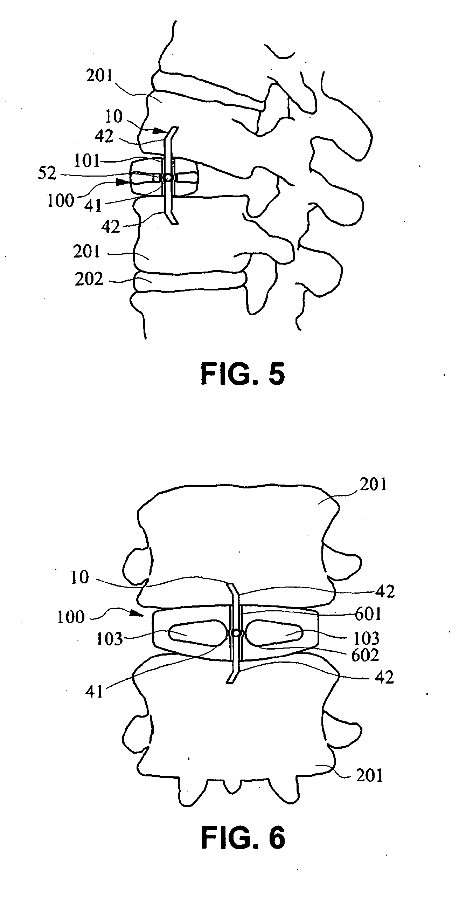

**Fig. 5** shows the side view of the bended (**42**) trans-vertebral and intra-vertebral plate (**10**) and the fusion cage (**100**) which are inserted from the lateral side of the spinal column. The cage has a vertical slot (**101**) to accommodate the plate (**10**). At the center of the trans-vertebral and intra-vertebral plate of the second preferred embodiment has a screw hole (**41**), and also at the center of the lateral end of the cage has a threaded screw hole (**52**) for the screw which is passing through the screw hole (**41**) of the plate (**10**).

**Fig. 6** shows a front view of the fusion cage (**100**) with an anterior vertical slot and the second preferred embodiment of the trans-vertebral and intra-vertebral plate (**10**) that they are inserted into the intervertebral space from the anterior side of the spinal column, in contrast to **Fig. 5****,** that the cage and the plate are inserted from the lateral side of the spinal column. The anteriorly inserted cage also has a vertical slot (**601**) for the plate and a screw hole (**602**) for the second preferred embodiment of the plate (**10**).

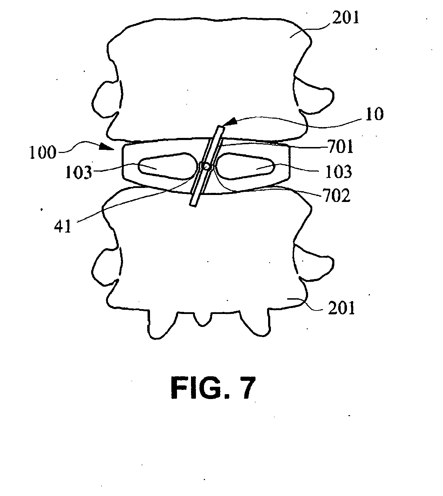

**Fig. 7** shows another anterior inserted cage and the trans-vertebral and intra-vertebral plate of the present invention. The fusion cage (**100**) has an anterior oblique slot (**701**) for the straight trans-vertebral and intra-vertebral plate (**10**). The straight plate has a screw hole (**41**) at the center of its out end and the cage has a corresponding threaded screw hole (**702**) for the screw which is inserted through the screw hole of the plate (**41**) to integrate the cage with the plate.

It is noteworthy to point out that the aforementioned preferred embodiments are used for illustrating the present invention only, but not intended to limit the scope of the present invention.

### Procedure

To insert the trans-vertebral and intra-vertebral plate (**10**) and the slotted fusion cage (**100**) fixation device from the lateral side of the lumbar column (with reference to **Fig. 2** and **Fig. 3**), the patient is placed on the right or left decubitus position, retroperitoneal space is entered from the left or right flank of the abdomen, after dissecting abdominal muscles. The psoas muscle covering the lateral surface of the disc is dissected posteriorly to expose the lateral surface of the disc. The disc material is then cleaned out until bony endplates are remained. The fusion cage (**100**) filled with bone graft is then inserted inside the prepared disc space from the lateral side of the disc space. A vertical shallow bone cut is made on the lateral surfaces of two adjacent vertebrae using an osteotome, through the vertical slot (**101**) of the cage. The length of the bone cut equals to the length of the longer end of the plate (**10**). The plate is grasped using a suitable tool; the thinned end (**11**) of the plate is perpendicularly facing the bone cut and gently hammering the plate inside the two adjacent vertebral bodies. The cage also can be made of having a screw hole (**52**) to lock the plate which being made of having a corresponding hole (**41**) in the middle of longer end of the plate to accommodate a screw. For the thoracic spine, the procedure is similar.

To insert the plate (**10**) and the fusion cage (**100**) from the anterior side of the spinal column (with reference to **Fig. 6** and **Fig. 7**), in the lumbar region, the patient is placed on supine position, and the anterior surface of the lumbar disc is exposed using the standard anterior approach method. After the disc space is prepared, the fusion cage (**100**) is inserted into the disc space from the anterior side of the disc space, a bone cut is made through the vertical slot (**601**) or the oblique slot (**701**) of the cage, and the plate is then hammered inside the vertebrae through the slot of the cage and the bone cut. After the cage and the plate are in place a screw is now inserted through the hole (**41**) of the plate and threaded into the corresponding screw hole (**602, 702**) of the cage and tightened. In the thoracic and cervical regions, the procedure is similar.

In summation of the description above, the trans-vertebral and intra-vertebral plate device (**10**) and the rectangular cage (**100**) with a slot (**101**) for the plate relates to interbody fusion, and brings forward the following advantages to the patients:
1. The trans-vertebral and intra-vertebral plate device is extremely easy for the surgeons to apply, which may reduce operation time and blood loss.
2. There is a relatively wide margin of safety because the trans-vertebral and intra-vertebral plate device has zero profile.
3. The trans-vertebral and intra-vertebral plate and the fusion cage each plays its own role of restricting intervertebral movement. When applied on the lateral side of the spinal column, the plate limits intervertebral movement of extension-flexion and axial rotation, and the cage limits intervertebral movement of flexion and lateral bending; when applied on the anterior side of the spinal column, the bended plate or obliquely inserted plate limits intervertebral movement of axial rotation and lateral bending, and extension. In addition, the combination of the plate and the cage using a screw locking mechanism may further reduce the possibility of dislodgement of the plate or the cage.
4. Compared to the screw based systems, there is relatively little risk of loosening of the plate device. Unlike spinal screws which often become loosened in osteoporotic spine.
5. In case the implanted plate device has to be removed, it is relatively easy to do so.

In summation of the above description, the trans-vertebral and intra-vertebral plate fixation device (**10**), and the cage (**100**) with a slot (**101**) for the plate of the present invention herein achieve the same purpose of three-dimensional fixation of current bone screw based fixation system and without bulky design, complicated assembling procedure and minimize the possible hazards to the human body, further complies with the patent application requirements, and thus is duly filed for patent application.

While the invention has been described by means of specific embodiments, numerous modifications and variations could be made thereto by those skilled in the art without departing from the scope of the invention set forth in the claims.

## Claims

1. A trans-vertebral and intra-vertebral plate and fusion cage device for spinal interbody fusion comprising a plate element (10) and a rectangular intervertebral fusion cage member (100) having a slot (101) at one of its lateral sides to accomodate said plate (10);
**characterized in that**,
the dimension of said slot (101) of said cage (100) is corresponding to the cross section of said plate (10) such that one of the thin elongated sides (11) of said plate (10) is facing cortical bones of two adjacent vertebrae (201) and said plate (10) is perpendicular to the cortical bones of said two adjacent vertebrae, said thin elongated side (11) being further thinned in order to facilitate the penetration of the plate into the two adjacent vertebra (202) and said slot (101), thereby burying the plate element (10) inside the two adjacent vertebrae (201) from lateral side or anterior side of adjacent vertebrae (201); said plate (10) and said cage (100) are coupled in such a manner that said plate (10) is trans-vertebral and intra-vertebral and said cage (100) is intervertebral, to achieve three-dimensional spinal interbody fixation.

2. The trans-vertebral and intra-vertebral plate and fusion cage device according to claim 1,
**characterized in that**,
said slot (101) for said plate (10) is vertical.

3. The trans-vertebral and intra-vertebral plate and fusion cage device according to claim 1,
**characterized in that**,
said slot (101) for said plate (10) is oblique.

4. The trans-vertebral and intra-vertebral plate and fusion cage device according to claim 1,
**characterized in that**,
the distal ends of said plate (10) are bended.

5. The trans-vertebral and intra-vertebral plate and fusion cage device according to one of the previous claims,
**characterized in that**
the center of outer elongated end of said plate (10) further comprises a protruded round hole (41) with a predetermined dimension, and at the center of said slot (101,701) of said cage (100) having a corresponding hole (52, 602, 702) configured for receiving said hole (41) of said plate (10), thereby said plate (10) and said cage (100) are integrated.

6. The trans-vertebral and intra-vertebral plate and fusion cage device according to one of the previous claims,
**characterized in that**
the surface of the plate (10) is perforated.

7. The trans-vertebral and intra-vertebral plate and fusion cage device according to one of the previous claims,
**characterized in that**
the cage (100) has holes (103).

8. The trans-vertebral and intra-vertebral plate and fusion cage device according to one of the previous claims,
**characterized in that**
the plate (10) is serrated.

## Patentansprüche

1. Transvertebrale und intravertebrale Platten- und Fusionskäfig-Vorrichtung zur interkorporellen Wirbelsäulenfusion, umfassend ein Plattenelement (10) und ein rechteckiges intervertebrales Fusionskäfig-Element (100) mit einem Schlitz (101) auf einer seiner lateralen Seiten, um die Platte (10) aufzunehmen;
**dadurch gekennzeichnet, dass**
die Dimension des Schlitzes (101) des Käfigs (100) dem Querschnitt der Platte (10) derart entspricht, dass eine der dünnen länglichen Seiten (11) der Platte (10) der Kortikalis von zwei angrenzenden Wirbeln (201) gegenübersteht und die Platte (10) zu der Kortikalis der beiden angrenzenden Wirbel rechtwinklig ist, wobei die dünne längliche Seite (11) ferner verdünnt ist, um das Eindringen der Platte in die beiden angrenzenden Wirbel (201) und den Schlitz (101) zu ermöglichen, wodurch das Plattenelement (10) im Innern der beiden angrenzenden Wirbel (201) von der lateralen Seite oder der anterioren Seite der angrenzenden Wirbel (201) verlegt wird; wobei die Platte (10) und der Käfig (100) derart gekoppelt sind, dass die Platte (10) transvertebral und intravertebral ist und der Käfig (100) intervertebral ist, um eine dreidimensionale interkorporelle Wirbelsäulenfixierung zu erreichen.

2. Transvertebrale und intravertebrale Platten- und Fusionskäfig-Vorrichtung nach Anspruch 1,
**dadurch gekennzeichnet, dass**
der Schlitz (101) für die Platte (10) senkrecht ist.

3. Transvertebrale und intravertebrale Platten- und Fusionskäfig-Vorrichtung nach Anspruch 1,
**dadurch gekennzeichnet, dass**
der Schlitz (101) für die Platte (10) schräg ist.

4. Transvertebrale und intravertebrale Platten- und Fusionskäfig-Vorrichtung nach Anspruch 1,
**dadurch gekennzeichnet, dass**
die distalen Enden der Platte (10) umgebogen sind.

5. Transvertebrale und intravertebrale Platten- und Fusionskäfig-Vorrichtung nach einem der vorhergehenden Ansprüche,
**dadurch gekennzeichnet, dass**
die Mitte eines äußeren länglichen Endes der Platte (10) ferner ein vorstehendes rundes Loch (41) einer vorbestimmten Dimension umfasst, und wobei in der Mitte des Schlitzes (101, 701) der Käfig (100) ein entsprechendes Loch (52, 602, 702) aufweist, das konfiguriert ist, um das Loch (41) der Platte (10) aufzunehmen, wodurch die Platte (10) und der Käfig (100) integriert sind.

6. Transvertebrale und intravertebrale Platten- und Fusionskäfig-Vorrichtung nach einem der vorhergehenden Ansprüche,
**dadurch gekennzeichnet, dass**
die Oberfläche der Platte (10) perforiert ist.

7. Transvertebrale und intravertebrale Platten- und Fusionskäfig-Vorrichtung nach einem der vorhergehenden Ansprüche,
**dadurch gekennzeichnet, dass**
der Käfig (100) Löcher (103) aufweist.

8. Transvertebrale und intravertebrale Platten- und Fusionskäfig-Vorrichtung nach einem der vorhergehenden Ansprüche,
**dadurch gekennzeichnet, dass**
die Platte (10) geriffelt ist.

## Revendications

1. Dispositif de plaque transvertébrale et intravertébrale et cage de fusion pour fusion rachidienne intercorporelle comprenant un élément de plaque (10) et un membre de cage de fusion intervertébrale rectangulaire (100) ayant une encoche (101) sur l'un de ses côtés latéraux pour loger ladite plaque (10);
**caractérisé en ce que**,
la dimension de ladite encoche (101) de ladite cage (100) correspondant à la section transversale de ladite plaque (10) de sorte que l'un des côtés fins allongés (11) de ladite plaque (10) se trouve en face des os corticaux des deux vertèbres adjacentes (201) et ladite plaque (10) est perpendiculaire aux os corticaux desdites deux vertèbres adjacentes (201), et ladite plaque (10) est perpendiculaire aux os corticaux desdites deux vertèbres adjacentes, ledit côté fin allongé (11) étant encore plus effilé pour faciliter la pénétration de la plaque dans les deux vertèbres adjacentes (201) et ladite encoche (101), enfouissant ainsi l'élément de plaque (10) à l'intérieur des deux vertèbres adjacentes (201) depuis un côté latéral ou un côté avant des vertèbres adjacentes (201) ; ladite plaque (10) et ladite cage (100) sont couplées d'une manière telle que ladite plapue (10) est transvertébrale et intravertébrale, et ladite cage (100) est intervertébrale, pour obtenir une fixation rachidienne intercorporelle tridimensionnelle.

2. Dispositif de plaque transvertébrale et intravertébrale et cage de fusion selon la revendication 1,
**caractérisé en ce que**,
ladite encoche (101) pour ladite plaque (10) est verticale.

3. Dispositif de plaque transvertébrale et intravertébrale et cage de fusion selon la revendication 1,
**caractérisé en ce que**,
ladite encoche (101) pour ladite plaque (10) est oblique.

4. Dispositif de plaque transvertébrale et intravertébrale et cage de fusion selon la revendication 1,
**caractérisé en ce que**,
les extrémités distales de ladite plaque (10) sont courbées.

5. Dispositif de plaque transvertébrale et intravertébrale et cage de fusion selon l'une quelconque des revendications précédentes,
**caractérisé en ce que**
le centre de l'extrémité extérieure allongée de ladite plaque (10) comprend en outre un trou rond qui dépasse (41) avec une dimension prédéterminée, et au centre de ladite encoche (101, 701) de ladite cage (100) ayant un trou correspondant (52, 602, 702) configuré pour recevoir ledit trou (41) de ladite plaque (10), ladite plaque (10) et ladite cage (100) sont ainsi intégrées.

6. Dispositif de plaque transvertébrale et intravertébrale et cage de fusion selon l'une quelconque des revendications précédentes,
**caractérisé en ce que**
la surface de la plaque (10) est perforée.

7. Dispositif de plaque transvertébrale et intravertébrale et cage de fusion selon l'une quelconque des revendications précédentes,
**caractérisé en ce que**
la cage (100) a des trous (103).

8. Dispositif de plaque transvertébrale et intravertébrale et cage de fusion selon l'une quelconque des revendications précédentes,
**caractérisé en ce que**
la plaque (10) est dentelée.
